# NEW EUROPEAN PATENT SPECIFICATION

(11) **EP 3 226 827 B2**
(45) Date of publication and mention of the opposition decision: **29.09.2021**
(45) Mention of the grant of the patent: 06.02.2019
(21) Application number: 15812897.5
(22) Date of filing: 01.12.2015
(51) Int. Cl.: A61K 8/20, A61K 8/34, A61K 8/39, A61Q 15/00

(54) **ALUMINIUM-FREE ANTIPERSPIRANT/DEODORANT COMPOSITIONS AND METHOD OF PREPARING THE SAME**
ALUMINIUMFREIE SCHWEISSHEMMENDE/DESODORIERENDE ZUSAMMENSETZUNG UND VERFAHREN ZUR HERSTELLUNG DAVON
COMPOSITIONS ANTI-TRANSPIRANTES/DÉODORANTES SANS ALUMINIUM ET PROCÉDÉ POUR LES PRÉPARER

(30) Priority: 05.12.2014 SE 1451492
(43) Date of publication of application: 11.10.2017
(73) Proprietor: Stockholms Analytiska Laboratorium AB, 134 40 Gustavsberg (SE)
(72) Inventor: WÅHLSTAM, René, 133 35 SALTSJÖBADEN (SE)
(74) Representative: Groth & Co. KB
(86) International application number: PCT/SE2015/051289
(87) International publication number: WO 2016/089288

(56) References cited:
- EP-A1- 1 588 690
- EP-A1- 2 604 249
- DE-A1-102011 088 967
- US-A1- 2014 271 517
- DATABASE GNPD [Online] MINTEL; August 2004 (2004-08), "Deodorant", XP002753564, Database accession no. 292548

## Description

### FIELD OF INVENTION

The present invention relates to an antiperspirant/deodorant composition which is effective for reducing perspiration and preventing or eliminating body malodours. In particular the present invention relates to an antiperspirant/deodorant composition that is essentially free of aluminium cations.

### BACKGROUND

There are two types of sweat glands, eccrine and apocrine glands. Eccrine glands are present all over the body and the secretion of eccrine glands comprises sodium chloride, urea, glucose, potassium and lactate ions, and approximately 99% of water. Thus the secretion of eccrine glands is the main cause of wet feeling on the skin. Apocrine glands are present in the axillae and urogenital regions of the body and the secretion of apocrine glands comprises fatty amino acids, cholesterol and various steroids. It is well known that malodurs develop as a result from microbial interactions with the secretion of sweat glands which subsequently produce malodour compounds, such as 3-methyl-2-hexenoic acid and other small chain fatty acids.

A deodorant reduces and/or masks malodurs through deodorant actives such as antiperspirant agents, antimicrobial agents, and/or fragrances. An antiperspirant inhibits perspiration by actively interfering with the secretion of sweat glands. Thus, an antiperspirant is, by effect, automatically a deodorant.

Conventional antiperspirant/deodorant products typically use aluminium-based salts as the active ingredients to control perspiration and malodour. The most frequently used antiperspirant aluminium salts include aluminium hydroxychloride, aluminium bromhydrate, aluminium sulphate, activated aluminium chlorohydrate (ACH) and activated aluminium zirconium chlorohydrate (AZCH). It is widely accepted that the antiperspirant action is achieved by diffusion of the soluble and acid aluminium salts into the sweat duct which is coupled with subsequent slow neutralization. The reaction takes place at or below the natural physiological pH of the skin surface, which produces a gelatinous and insoluble polymeric aluminium hydroxide-protein gel that partially blocks the orifices of the sweat glands whereby the flow of sweat is reduced to a certain degree although not eliminated completely. Furthermore the aluminium salts have astringent effects on skin, tightening the sweat glands which are further narrowed. Aluminium salts present in the conventional antiperspirant/deodorant compositions may also act as antimicrobial agents that enhance the deodorant effects of the compositions by reducing the odour-causing microbial population on the skin.

Aluminium chlorohydrate has been established as a neurotoxin. At higher doses aluminium as such is capable of causing DNA damage, and adversely affects the blood-brain barrier. Small amounts of aluminium can be absorbed from the gastrointestinal tract and through the skin. Although the health impact of aluminium on human body is disputed, many consumers may hold a negative view on the use of antiperspirant/deodorant products containing aluminium salts. The application of aluminium salts may lead to skin irritation since some people are allergic to aluminium and may develop contact dermatitis when exposed to those products. Aluminium salts may also react with sweat to create irreversible stains on clothing.

Alternative antiperspirant/deodorant compositions without aluminium salts are available and some rely on the use of specifically developed antimicrobial agents, such as triclosan. However, there are health and environmental concerns with using these antimicrobial agents. There is evidence suggesting that triclosan is an endocrine disrupter and it may cause cross-resistance in microorganisms. Triclosan is also a chlorinated organic compound which may accumulate in the environment. Natural substances extracted from plants, such as essential oil from *salvia officinalis* or usnic acid from *usnea barbata,* has been used as active ingredients in the aluminium-free antiperspirant/deodorant compositions. However, these alternative compositions are generally much less effective in controlling perspiration than conventional compositions containing aluminum salts. Other approaches for controlling body malodors include using odour-absorbing agents such as carbonate and bicarbonate salts, activated charcoal and zeolites. However, these odour-absorbing agents have little effects on perspiration and become ineffective when wet.

Accordingly, there is a need for novel antiperspirant/deodorant compositions with a significantly reduced or even zero level of aluminum which have uncompromised antiperspirant/deodorant efficacy.

It is therefore an object of the present invention to provide an essentially aluminium-free antiperspirant/deodorant composition which is at least as efficient as conventional antiperspirant/deodorant compositions containing aluminium salts in terms of controlling perspiration and preventing or eliminating body odours.

It is a further object of the present invention to provide such a composition which does not have the problems associated with conventional antiperspirant/deodorant compositions containing aluminium salts.

### SUMMARY OF THE INVENTION

The present invention is directed to an aluminium-free antiperspirant/ deodorant composition according to claim 1 comprising, based on the total weight of the composition 0 % by weight of aluminium cations.

The composition comprises, based on the total weight of the composition, at least 5 % by weight of one or more ionizable halide salts soluble in water, wherein the one or more halide salts are antimicrobial agents selected from the group consisting of sodium chloride and potassium chloride. The concentration of the one or more halide salts is 5 - 35 %, preferably 5 - 25 %, and more preferably 10 - 25 % by weight based on the total weight of the composition. Sodium and potassium chlorides are natural ingredients in table salts that are able to inhibit growth of many bacteria when used in high concentration.

The composition further comprises one or more glycols or polyglycols. The concentration of the one or more glycols or polyglycols is 0.5 - 50 %, preferably 1 - 15 % by weight of the total composition. The one or more glycols or polyglycols are selected from the group consisting of glycerol; diglycerol; trimethylene glycol; tetramethylene glycol; ethylene glycol; diethylene glycol; triethylene glycol; tetraethylene glycol; dipropelene glycol; tripropelene glycol; butylene glycol; 1,3-butanediol; methyl propanediol; pentylene glycol; hexamethylene glycol; cyclohexanedimethanol; 2,2,4,4-tetramethyl-1,3-cyclobutanediol; neopentyl glycol; PEG-4 through 100; PPG-9 through 34. Preferably the glycol is trimethylene glycol. Trimethylene glycol is a commercially available astringent that may cause contraction of sweat glands, which enhances the antiperspirant effect.

The concentration of the one or more cationic species may be 0.01 - 10 %, preferably 0.05 - 0.5 % by weight based on the total weight of the composition. The cationic species is preferably a quaternary ammonium polymer selected from the group consisting of polyquaternium-1, 2, 4 through 20, 22, 24, 27 through 37, 39, 42 through 47. More preferably the cationic species is polyquaternium-47 or polyquaternium-7. The positive charges of a polyquaternium improve its film-forming properties by strengthening the ionic bonding to skin, and thereby help deposit other components on the skin.

The composition has a pH range of 2 to 8, preferably 2 to 5 or 7 to 8, more preferably 2.8 to 3.5, and even more preferably a pH of approximately 3. Synergistic antiperspirant and deodorant effects are accomplished by a combination of the water-soluble ionizable halide salts, the glycols or polyglycols and the water-soluble nonmetal cationic species.

The present invention is also directed to a cosmetic preparation comprising the essentially aluminium-free antiperspirant/deodorant composition according to any one of the claims 1 to 11 for skin care, in particular for treating axillae or feet.

The present invention is further directed to a process for preparing the essentially aluminium-free antiperspirant/deodorant composition according to any one of the claims 1 to 11.

The present invention is further directed to use of the essentially aluminium-free antiperspirant/deodorant composition according to any one of the claims 1 to 11 for preparing a cosmetic preparation for skin care.

It has been found that the essentially aluminium-free antiperspirant/deodorant compositions according to the present invention have at least comparable antiperspirant and/or deodorant effects compared with conventional antiperspirant/deodorant products containing aluminium salts. The compositions according to the present invention are mild to the skin and do not have the problems associated with conventional antiperspirant/deodorant products containing aluminium salts.

### BRIEF DESCRIPTION OF THE DRAWINGS

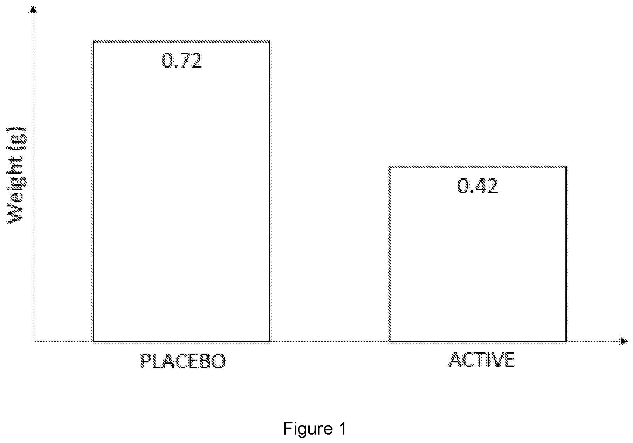

Figure 1 illustrates that the amount of sweat measured from axillae treated with the active composition is significantly less than the placebo. Median value of the amount of sweat (g) excreted from axillae treated with the active composition is compared with the placebo.

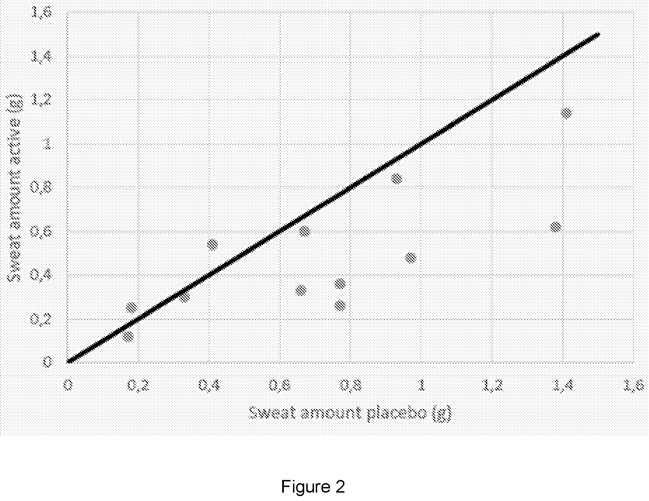

Figure 2 illustrates that 10 of 12 test subjects secreted less sweat for axillae treated with the active composition compared to the placebo. Each dot in the graph represents a single subject describing the amount of sweat (g) measured for an axilla treated with the active composition. The inserted line represents the fictive points where the amount of sweat would be the same for axillae treated with the active composition and with the placebo. A dot under the line means that less sweat was measured for an axilla treated with the active composition.

### DETAILED DESCRIPTION OF THE INVENTION

The term "active ingredients" refers to the ingredients responsible for the antiperspirant or deodorant effects of the composition according to the present invention.

The term "aluminium-free" or "essentially aluminium-free" refers to a concentration of less than 3 %, more preferably 0 % by weight of aluminium cations, based on the total weight of the composition.

The term "odour" or "malodour" is used to mean odours generated as a result of natural functioning of a human or mammalian body. Such odours include, but not limited to, odours produced by microbial decomposition of skin secretions. Such odour compounds are mainly organic molecules with different structures and functional groups, such as amines, acids, alcohols, aldehyde, ketones, phenolics, and polycyclics including aromatics and polyaromatics. These compounds may also comprise sulfur-containing functional groups, such as thiol, mercaptan, sulfide and/or disulfide groups.

The term "deodorant effects" means an activity of substantially reducing or eliminating unpleasant odours and more particularly body odours.

The term "antiperspirant effects" means an activity of substantially reducing secretions of the sweat glands.

The term "emulsion" refers to a mixture of two liquids that normally cannot be mixed, in which one liquid is dispersed in the other liquid as very fine droplets. Emulsifying agents are often used to help form the emulsion and stabilizing agents are used to keep the resulting emulsion from separating. The most common emulsions are oil-in-water emulsions (where oil droplets are dispersed in water) and water-in-oil emulsions (where water droplets are dispersed in oil).

### ACTIVE INGREDIENTS

It has been surprisingly found that high concentration of water-soluble ionizable halide salts significantly improves the antiperspirant/deodorant effects of available aluminium-free antiperspirant/deodorant compositions. This is attributed mainly to the antimicrobial activities of the halide salts by inducing hypertonic tension. When the external salt concentration is high enough water is withdrawn from microorganisms, which is fatal to the flora of bacteria present on skin and which also inhibits germination of spores.

However, high concentration of salts may also lead to withdrawn of water from human cells. This can be counteracted by addition of one or more glycols or polyglycols which have astringent effects. A combination of glycols or polyglycols with high concentration of salts has significantly improved antiperspirant/deodorant effects compared with available aluminium-free antiperspirant/deodorant compositions with or without high concentration of salts.

The antiperspirant/deodorant effects are further enhanced by a triple combination of water-soluble ionizable halide salts, glycols or polyglycols and water-soluble nonmetal cationic species which act synergistically. The cationic species tend to adhere to the anionic charges of the skin and form a continuous film that helps deposit other components of the composition on the skin. The composition of the present invention comprising the triple combination are at least as efficient as conventional antiperspirant/deodorant compositions with aluminium salts.

### Water-soluble ionizable halide salts

The composition of the present invention comprises, based on the total weight of the composition, at least 5 % by weight of one or more ionizable halide salts soluble in water selected from the group consisting of sodium chloride and potassium chloride which are antimicrobial agents. The concentration of the one or more halide salts selected from the group consisting of sodium chloride and potassium chloride is 5 - 35 %, preferably 5 - 25 %, and more preferably 10 - 25 % by weight of the total composition.

Sodium and potassium chlorides are natural ingredients in table salts that are able to inhibit growth of many bacteria when used in high concentration. They cause less irritation or uncomfortable sensation when applied to the skin compared with aluminium salts or other antimicrobial agents. Furthermore, the use of sodium and potassium chlorides in the composition of the present invention will not cause irreversible stains on clothing.

### Glycols or polyglycols

The composition further comprises one or more glycols or polyglycols. The concentration of the one or more glycols or polyglycols is 0.5 - 50 %, preferably 1 - 15 % by weight of the total composition. The one or more glycols or polyglycols are preferably selected from the group consisting of glycerol; diglycerol; trimethylene glycol; tetramethylene glycol; ethylene glycol; diethylene glycol; triethylene glycol; tetraethylene glycol; dipropelene glycol; tripropelene glycol; butylene glycol; 1,3-butanediol; methyl propanediol; pentylene glycol; hexamethylene glycol; cyclohexanedimethanol; 2,2,4,4-tetramethyl-1,3-cyclobutanediol; neopentyl glycol; PEG-4 through 100; PPG-9 through 34.

More preferably, the glycol is trimethylene glycol. Trimethylene glycol is a commercially available astringent that may cause contraction of sweat glands, which enhances the antiperspirant effect. Trimethylene glycol with excellent sensory characteristics does not cause skin irritation but rather improves skin moisturization. Since trimethylene glycol can boost the efficacy of preservatives, the amount of preservatives needed in the formulation may be reduced without adverse impacts on the shelf life of the composition of the present invention. Preferably, no preservative is present in the composition of the present invention.

### Water-soluble nonmetal cationic species

The concentration of the one or more cationic species may be 0.01 - 10 %, preferably 0.05 - 0.5 % by weight of the total composition. The cationic species is preferably a quaternary ammonium polymer selected from the group consisting of polyquaternium-1, 2, 4 through 20, 22, 24, 27 through 37, 39, 42 through 47. More preferably the cationic species is polyquaternium-47 or polyquaternium-7. The positive charges of a polyquaternium improve its film-forming properties by strengthening the ionic bonding to skin, and thereby help deposit other components on the skin.

In addition to the active ingredients described hereinbefore, the deodorant compositions of the present invention may further comprise one or more optional components which may modify the physical or chemical characteristics of the compositions or serve as additional "active" components when deposited on the skin, provided that the optional components are physically and chemically compatible with the essential components described herein, or do not otherwise unduly impair product stability, aesthetics, or performance. Preferred optional components are emollients, emulsifiers, solvents, thickeners, stabilizers, pH regulators and/or odour-absorbing agents.

### EMOLLIENTS

Emollients, also commonly referred to as moisturizers, are products that help to soften skin or to treat skin that has become dry. Most emollients are forms of oil or grease. They work by increasing the ability of the skin to hold water, providing the skin with a layer of oil to prevent water loss, and lubricating the skin. An emollient ingredient may also help other ingredients penetrate skin better.

Emollients useful herein include, but are not limited to, triglycerides such as vegetable oils (e.g., avocado oil, sunflower seed oil) or mineral oils; esters such as oleyl oleate, isopropyl palmitate, or isopropyl myristate; hydrocarbons such as liquid petrolatum comprising C16-C32 linear or branched hydrocarbon, liquid polyisobutylene, squalene, pristine paraffin; fatty acid higher alcohol such as oleyl alcohol; lanolin and its derivatives; phospholipids; PPG-15 stearyl ether. The composition of the present invention usually comprises 1-5 % by weight of an emollient.

A preferred emollient ingredient of the composition according to the present invention is dicaprylyl carbonate that has the ability to moisturize the skin, making it feel soft and smooth without leaving a greasy residue behind. It has excellent spreadability, and leaves a dry, velvety feel to the skin similar to how a silicone would. Furthermore, it emulsifies easily and, has long-term emulsion stability and facilitates the application of slow spreading ingredients.

### EMULSIFIERS

Emulsifiers are compounds used to help keep the various ingredients in the product from separating. The emulsifiers stabilize the combination of active and inactive ingredients and make them blend together smoothly.

The emulsifying agents typically used include ceteareth-20, cetearyl glucoside, ceteth-10, ceteth-2, ceteth-20, cetyl alcohol, cocamide MEA, glyceryl laurate, glyceryl stearate (and) PEG-100 stearate, glyceryl stearate, glyceryl stearate SE, glycol distearate, glycol stearate, isoceteth-20, isosteareth-20, laureth-23, laureth-4, lecithin, methyl glucose sesquistearate, oleth-10/polyoxyl 10 oleyl ether NF, oleth-10, oleth-2, oleth-20, PEG-100 stearate, PEG-20 almond glycerides, PEG-20 methyl glucose sesquistearate, PEG-25 hydrogenated castor oil, PEG-30 dipolyhydroxystearate, PEG-4 dilaurate, PEG-40 sorbitan peroleate, PEG-60 almond glycerides, PEG-7 olivate, PEG-7 glyceryl cocoate, PEG-8 dioleate, PEG-8 laurate, PEG-8 oleate, PEG-80 sorbitan laurate, polysorbate 20, polysorbate 20 NF, polysorbate 60, polysorbate 60 NF, polysorbate 80, polysorbate 80 NF, polysorbate 85, PPG-11 stearyl ether, propylene glycol isostearate, sorbitan isostearate, sorbitan laurate, sorbitan monostearate NF, sorbitan oleate, sorbitan sesquioleate, sorbitan stearate (and) sucrose cocoate, sorbitan stearate, sorbitan trioleate, stearamide MEA, steareth-100, Steareth-2, steareth-20, steareth-21 and silicone-based emusifiers.

Preferably, a combination of steareth-2 and steareth-21 is used as emulsifiers for the composition of the present invention. They are mild, virtually odorless nonionic surfactants. Steareth-2 has an HLB of 4.9 and is used at 2-5 % (w/w). Steareth-21 is a waxy solid with an HLB of 15.5 and is typically used at 1-3 % (w/w).

### SOLVENTS AND DILUENTS

Solvents used in the present invention include ethanol, water and water solutions of lower alkyl alcohols. Lower alkyl alcohols useful herein are monohydric alcohols having 1 to 6 carbons, more preferably ethanol and isopropanol.

Preferably, the solvent is substantially water. Deionized water is more preferably used. Water from natural sources including mineral cations can also be used, depending on the desired characteristic of the product.

The solvent will typically amount to from about 50% to about 90% by weight based on the total weight of the composition of the present invention. Preferably the composition of the present invention comprises the solvent from about 60% to about 80% by weight based on the total weight of the composition.

### THICKENERS AND STABILIZERS

A thickener is employed to impart to the composition the appropriate flow properties suited to the type of applicator. Typical thickeners include natural gums such as tragacanth, gum acacia, locust bean gum, or synthetic gums such as hydroxymethyl cellulose, hydroxypropyl cellulose, methylcellulose, carboxymethyl cellulose, carboxy vinyl polymer and starch. Some inorganic substances, such as pyrogenic silica, precipitated silicas or clays, such as Bentonite can be used to thicken the compositions. Also, alginates or polyvinyl pyrollidone, sodium stearate or other soaps can be used for this purpose.

The preferred thickener selected for the composition of the present invention is tapioca starch polymethylsilse at a concentration of 0,5-5 % (w/w).

### pH REGULATORS

The composition has a pH range of 2 to 8, preferably 2 to 5 or 7 to 8, more preferably 2.8 to 3.5, and a pH value of approximately 3 is even more preferred. Buffers and other pH adjusting agents including i.a. citric acid, acetic acid and ethyl lactate, can be added into the composition to achieve the desirable pH value. The preferred pH regulator for the composition of the present invention is citric acid.

According to the present invention, the cosmetic antiperspirant/deodorant products for skin care may be formulated in a variety of product forms, such as solid sticks, creams, soft-solids, roll-on lotions, aerosols, pump sprays, squeeze sprays, solutions, ointments, gels or emulsions.

A formulation (I) of particular interest comprises the following ingredients as detailed in Table 1.

**Table 1**

| Ingredient | % (w/w) |
|---|---|
| dicaprylyl carbonate | 1-5 |
| steareth-2 | 2-5 |
| steareth-21 | 1-3 |
| water | for use as solvent |
| polyquaternium-47 | 0.01-1 |
| trimethylene glycol | 2-15 |
| sodium chloride | 5-20 |
| citric acid | for adjustment to pH 2 to 5 |
| tapioca starch polymethylsilse | 0.5-5 |
| ∑. | 100 |

The method of preparing the preferred composition comprises the following steps:
- add the water-soluble components including sodium chloride, trimethylene glycol, polyquaternium-47 and citric acid into the water to make a first mixture;
- heat the first mixture to 75 °C;
- mix the fat components including dicaprylyl carbonate, steareth-2 and steareth-21 into a second mixture;
- heat the second mixture to 80 °C;
- add the second mixture into the heated first mixture to make a third mixture;
- the third mixture is allowed to emulsify by stirring;
- the emulsion is allowed to cool off to 40 °C;
- tapioca starch polymethylsilse is added into the emulsion;
- the final emulsion is allowed to cool off to 30 °C.

The following examples further describes and demonstrate embodiments within the scope of the present invention. The examples are given solely for the purpose of illustration and are not to be construed as limitations of the present invention, as many variations thereof are possible without departing from the scope of the invention.

### Example 1 (reference example)

A composition is prepared by mixing the following ingredients as detailed in Table 1.1.

**Table 1.1**

| Ingredient | % (w/w) |
|---|---|
| dicaprylyl carbonate | 3 |
| steareth-2 | 4 |
| steareth-21 | 2 |
| water | 73.95 |
| sodium chloride | 15 |
| citric acid | 0.05 |
| tapioca starch polymethylsilse | 2 |
| ∑. | 100 |

### Example 2 (ref)

A reference composition is prepared by mixing the following ingredients as detailed in Table 1.2.

**Table 1.2**

| Ingredient | % (w/w) |
|---|---|
| dicaprylyl carbonate | 3 |
| steareth-2 | 4 |
| steareth-21 | 2 |
| water | 71.95 |
| trimethylene glycol | 2 |
| sodium chloride | 15 |
| citric acid | 0.05 |
| tapioca starch polymethylsilse | 2 |
| ∑. | 100 |

### Example 3 (ref)

A reference composition is prepared by mixing the following ingredients as detailed in Table 1.3.

**Table 1.3**

| Ingredient | % (w/w) |
|---|---|
| dicaprylyl carbonate | 3 |
| steareth-2 | 4 |
| steareth-21 | 2 |
| water | 63.95 |
| trimethylene glycol | 10 |
| sodium chloride | 15 |
| citric acid | 0.05 |
| tapioca starch polymethylsilse | 2 |
| ∑. | 100 |

### Example 4 (ref)

A reference composition is prepared by mixing the following ingredients as detailed in Table 1.4.

**Table 1.4**

| Ingredient | % (w/w) |
|---|---|
| dicaprylyl carbonate | 3 |
| steareth-2 | 4 |
| steareth-21 | 2 |
| water | 65.95 |
| trimethylene glycol | 5 |
| sodium chloride | 17 |
| citric acid | 0.05 |
| tapioca starch polymethylsilse | 3 |
| ∑. | 100 |

### Example 5

A composition according to the present invention is prepared by mixing the following ingredients as detailed in Table 1.5.

**Table 1.5**

| Ingredient | % (w/w) |
|---|---|
| dicaprylyl carbonate | 3 |
| steareth-2 | 4 |
| steareth-21 | 2 |
| water | 65.85 |
| polyquaternium-47 | 0.1 |
| trimethylene glycol | 5 |
| sodium chloride | 17 |
| citric acid | 0.05 |
| tapioca starch polymethylsilse | 3 |
| ∑. | 100 |

### Alu Deo

An antiperspirant/deodorant composition comprising an aluminium salt is prepared by mixing the following ingredients as detailed in Table 1.6. This composition is designated as Alu Deo serving as a control.

**Table 1.6**

| Ingredient | % (w/w) |
|---|---|
| PPG-15 stearyl ether | 4.69 |
| steareth-2 | 2.06 |
| steareth-21 | 0.94 |
| persea gratissima oil | 1.41 |
| tocopheryl acetate | 0.19 |
| water | 76.29 |
| aluminum chlorohydrate | 14.00 |
| aloe barbadensis leaf juice | 0.23 |
| hydrogenated castor oil | 0.19 |
| ∑. | 100 |

Each axilla of any one test subject at each test occasion was treated differently as indicated in Table 1.7 to 1.10, which allows comparison of the effects of two different treatments on one and the same test subject.

### Antiperspirant test

Antiperspirant test was performed by measuring the axillary sweat weight in gram (g) produced by the test subjects #1 to 6 after the compositions of Example (Ex.) 1 to 6 were applied to them. Where the axillary sweat weight of less than 0.1 g were produced by each of the both axillae the values are not considered for comparison studies due to relatively high risk for deviation.

### Sniff test

Sniff test was carried out to determine the deodorant effect of the compositions of Ex. 1 to 6. Body odour was evaluated on a scale of 1 to 7, where 1 represents weak and 7 a strong body odour.

In a first experiment Ex. 1 was applied to one axilla while Ex. 2 or Ex. 3 to the other axilla of each test subject. The test subjects were subsequently allowed to perform daily duties before the antiperspirant and sniff tests. The results presented in Table 1.7 illustrate that Ex. 3 comprising 15 % (w/w) NaCl + 10 % (w/w) trimethylene glycol has enhanced antiperspirant performance on three of the four test subjects compared with Ex. 1 comprising 15 % (w/w) NaCl without trimethylene glycol. Ex. 3 has also enhanced deodorant effect on two test subjects, #1 and #4, compared with Ex. 1. On the other hand Ex. 2 comprising 15 % (w/w) NaCl and 2 % (w/w) trimethylene glycol has no obvious advantage over Ex. 1 with regard to either antiperspirant performance or deodorant effect.

In a second experiment Ex. 3 was applied to one axilla while leaving the other axilla untreated. The test subjects were subsequently allowed to work out on a treadmill to consume 200 kcal. The results presented in Table 1.8 illustrate that Ex. 3 was able to significantly reduce the production of axillary sweat even in a situation when the test subjects were under extreme stress.

In a third experiment Ex. 4 comprising 17 % (w/w) NaCl + 5 % (w/w) trimethylene glycol was compared with Ex. 5 comprising 17 % (w/w) NaCl + 5 % (w/w) trimethylene glycol + 0.1 % (w/w) polyquaternium-47 on the same condition as in the first experiment. The results presented in Table 1.9 illustrate that the test subject responded significantly better to Ex. 5 than Ex. 4 in respect of antiperspirant performance. However, the deodorant effect is comparable between Ex. 4 and Ex. 5.

In a fourth experiment Ex. 5 was compared with Alu Deo comprising the conventional antiperspirant agent aluminium chlorohydrate at a concentration of 14 % (w/w). The results presented in Table 1.10 illustrate that the essentially aluminium-free Ex. 5 outperforms Alu Deo in respect of both antiperspirant performance and deodorant effect.

**Table 1.7**

| Test | Test subject | Example 1 | Example 2 | Example 3 | Perspiration reduction compared with Ex. 1 | |
|---|---|---|---|---|---|---|
| Antiperspirant | #1 | 0.19 | n/a | 0.03 | -84 % | avg. -62 % |
| | | 0.46 | n/a | 0.28 | -39 % | |
| | | 0.3 | n/a | 0.11 | -63 % | |
| | | 0.04 | 0.01 | n/a | - | - |
| | #2 | 0.04 | n/a | 0.09 | - | avg. -28 % |
| | | 0.18 | n/a | 0.13 | -28 % | |
| | #3 | 0.45 | n/a | 0.73 | -62 % | avg. -40 % |
| | | 0.67 | n/a | 0.55 | -18 % | |
| | #4 | 0 | n/a | 0 | - | - |
| | #5 | 0.01 | 0.02 | n/a | - | - |
| Sniff | #1 | 7 | n/a | 1 | | |
| | | 1 | n/a | 2 | | |
| | | 4 | n/a | 4 | | |
| | | 1 | 1 | n/a | | |
| | #2 | 2 | n/a | 3 | | |
| | | 2 | n/a | 1 | | |
| | #3 | 8 | n/a | 4 | | |
| | | 2 | n/a | 6 | | |
| | #4 | 3 | n/a | 1 | | |
| | #5 | 1 | 1 | n/a | | |

**Table 1.8**

| Test subject | No treatment | Example 3 | Perspiration reduction compared with no treatment | |
|---|---|---|---|---|
| #1 | 1.64 | 1.45 | -12 % | avg. -19 % |
| | 1.93 | 1.44 | -25 % | |
| #6 | 0.31 | 0.07 | -77 % | avg. -58 % |
| | 0.18 | 0.11 | -39 % | |

**Table 1.9**

| Test | Test subject | Example 4 | Example 5 | Perspiration reduction compared with Ex. 4 | |
|---|---|---|---|---|---|
| Antiperspirant | #1 | 0.36 | 0.24 | -33 % | avg. -14 % |
| | | 0.44 | 0.46 | 5% | |
| Sniff | #1 | 3 | 3 | | |
| | | 3 | 3 | | |

**Table 1.10**

| Test | Test subject | Alu Deo | Example 5 | Perspiration reduction compared with Alu Deo |
|---|---|---|---|---|
| Antiperspirant | #2 | 0.02 | 0.02 | - |
| | #3 | 0.1 | 0.05 | -50 % |
| | #4 | 0.04 | 0.06 | - |
| | | 0.01 | 0.01 | - |
| | #5 | 0.01 | 0.02 | - |
| | | 0.04 | 0.03 | - |
| Sniff | #2 | 3 | 1 | |
| | #3 | 3 | 0 | |
| | #4 | 2 | 2 | |
| | | 0 | 0 | |
| | #5 | 2 | 1 | |
| | | 1 | 0 | |

A formulation (II) of particular interest comprises the following ingredients as detailed in the left column of Table 2. The placebo contains preservatives to prevent microbial growth. The formulation (II) was prepared in the same manner as the formulation (I).

**Table 2**

| Ingredient | |
|---|---|
| ACTIVE (pH 8.0) | PLACEBO (pH 7.9) |
| water | water |
| sodium chloride | - |
| trimethylene glycol | - |
| steareth-2 | steareth-2 |
| sodium bicarbonate | - |
| tapioca starch polymethylsilsesquioxane | tapioca starch polymethylsilsesquioxane |
| dicaprylyl carbonate | dicaprylyl carbonate |
| steareth-21 | steareth-21 |
| isododecane | isododecane |
| neopentyl glycol diheptanoate | neopentyl glycol diheptanoate |
| polyquaternium-7 | polyquaternium-7 |
| - | preservatives: phenoxyethanol, methylparaben, propylparaben, butylparaben, and isobutylparaben |

The active composition and the placebo as shown in Table 2 were randomized for use under the left and right axilla respectively. The samples were labeled with subject number and L for left and R for right. Excel was used for randomization. The randomization scheme was handled and stored by the person responsible for the labeling of products and were hold separated from both test leader and test subjects throughout the study. The study was thus double-blind, i.e. neither subjects nor the test leader knew which preparation was applied under each axilla. The products were supplied to test subjects along with instructions for use and information about the study layout. The test subjects applied the products themselves in the morning for five consecutive days. The subjects were instructed to apply plenty of product. They were not allowed to use any other antiperspirant or deodorant under the axillae while the experiments took place. They could use their normal soap and water for cleansing. The product efficacy was evaluated on the fifth day of application, about 8 hours after last application. The subjects were not allowed to wash their axillae between the last application and evaluation.

### Evaluation of scent

The deodorant effect of the compositions was evaluated by the test subjects themselves on a modified visual analogue scale (VAS) ranging from 0 % to 100 % where 0 % means very bad scent while 100% means very good natural neutral scent.

The subjects valued they had a significantly better smell under the axilla treated with the active composition than with the placebo (p < 0.01).

### Quantification of sweat secretion

Moisture-absorbing material was taped to the axillae of the subjects. The moisture-absorbing material and tape were weighed before application to the axillae. The subjects were then instructed to carry out light to moderate physical activity for at least 30 minutes (to stimulate sweat secretion). The moisture-absorbing material and tape were weighed again after the physical activity, and the difference in weight describes the perspiration taking place under each axilla during the physical activity. All weight measurements were made on an analytical balance with 2 decimal places.

The amount of sweat secreted from axillae treated with the active composition is significantly less than axillae treated with the placebo (p < 0.05). The reduction in amount of sweat, 8 hours after the last application using median value for the whole group, was 42% for treatment with the active composition compared to treatment with the placebo (see Figure 1). 10 of 12 subjects excreted less sweat from axillae treated with the active composition (see Figure 3).

## Claims

1. An aqueous antiperspirant/deodorant composition comprising, based on the total weight of the composition, 0 % by weight of aluminium cations, one or more glycols or polyglycols, and at least 5 % by weight of one or more ionizable halide salts soluble in water, wherein the one or more halide salts are antimicrobial agents, and wherein the composition has a pH range of 2 to 8, wherein the one or more halide salts are selected from the group consisting of sodium chloride and potassium chloride, wherein said composition further comprises one or more water-soluble non-metal cationic species.

2. A composition according to claim 1, wherein the concentration of the one or more halide salts is 5 - 35 %, preferably 5 - 25 %, and more preferably 10 - 25 % by weight of the total composition.

3. A composition according to any one of the claims 1 to 2, wherein the concentration of the one or more glycols or polyglycols is 0.5 - 50 %, preferably 1 - 15 % by weight of the total composition.

4. A composition according to any one of the claims 1 to 3, wherein the concentration of the one or more cationic species is 0.01 -10 %, preferably 0.05 - 0.5 % by weight of the total composition.

5. A composition according to any one of the preceding claims, wherein the one or more glycols or polyglycols are selected from the group consisting of glycerol; diglycerol; trimethylene glycol; tetramethylene glycol; ethylene glycol; diethylene glycol; triethylene glycol; tetraethylene glycol; dipropelene glycol; tripropelene glycol; butylene glycol; 1,3-butanediol; methylpropanediol; pentylene glycol; hexamethylene glycol; cyclohexanedimethanol; 2,2,4,4-tetramethyl-1,3-cyclobutanediol; neopentyl glycol; PEG-4 through 100; PPG-9 through 34.

6. A composition according to claim 5, wherein the glycol is trimethylene glycol.

7. A composition according to any one of the claims 1 to 6, wherein the one or more cationic species are quaternary ammonium polymers.

8. A composition according to claim 7, wherein the cationic species is polyquaternium-47.

9. A cosmetic preparation for skin care comprising the composition according to any one of the preceding claims.

10. A cosmetic preparation according to claim 9, wherein the cosmetic preparation is used for treating axillae.

11. A cosmetic preparation according to claim 9, wherein the cosmetic preparation is used for treating feet.

12. A process for preparing the composition according to any one of the claims 1 to 8, which process comprises the steps of
- adding water-soluble components into water to make a first mixture and heating the first mixture to 75 °C;
- mixing fat components into a second mixture and heating the second mixture to 80 °C;
- adding the second mixture into the heated first mixture to make a third mixture;
- allowing the third mixture to emulsify and cool off to 40 °C;
- optionally adding other ingredients into the emulsion; and
- allowing the final emulsion to cool off to 30 °C.

13. Use of the composition according to any one of the claims 1 to 8 for preparing a cosmetic preparation for skin care.

## Patentansprüche

1. Wässrige schweißhemmende/deodorierende Zusammensetzung, umfassend, auf Grundlage des Gesamtgewichts der Zusammensetzung, 0 Gew.-% Aluminiumkationen, ein oder mehrere Glykole oder Polyglykole und zumindest 5 Gew.-% eines ionisierbaren Halogenidsalzes oder mehrerer ionisierbarer Halogenidsalze, die in Wasser löslich sind, wobei das eine ionisierbare Halogenidsalz oder die mehreren ionisierbaren Halogenidsalze antimikrobielle Mittel sind und wobei die Zusammensetzung einen pH-Wert-Bereich von 2 bis 8 aufweist, wobei das eine ionisierbare Halogenidsalz oder die mehreren ionisierbaren Halogenidsalze aus der Gruppe ausgewählt sind, die aus Natriumchlorid und Kaliumchlorid besteht, wobei die Zusammensetzung ferner eine oder mehrere wasserlösliche NichtMetall-Spezies umfasst.

2. Zusammensetzung nach Anspruch 1, wobei die Konzentration des einen ionisierbaren Halogenidsalzes oder der mehreren ionisierbaren Halogenidsalze 5-35 Gew.-%, vorzugsweise 5-25 Gew.-% und noch bevorzugter 10-25 Gew.-% der Gesamtzusammensetzung beträgt.

3. Zusammensetzung nach einem der Ansprüche 1 bis 2, wobei die Konzentration des einen Glykols oder Polyglykols oder der mehreren Glykole oder Polyglykole 0,5-50 Gew.-%, vorzugsweise 1-15 Gew.-%, der Gesamtzusammensetzung beträgt.

4. Zusammensetzung nach einem der Ansprüche 1 bis 3, wobei die Konzentration der einen oder mehreren kationischen Spezies 0,01-10 Gew.-%, vorzugsweise 0,05-0,5 Gew.-%, der Gesamtzusammensetzung beträgt.

5. Zusammensetzung nach einem der vorangehenden Ansprüche, wobei das eine oder die mehreren Glykole oder Polyglykole aus der Gruppe ausgewählt sind, die aus Folgendem besteht: Glycerin; Diglycerin; Trimethylenglykol; Tetramethylenglykol; Ethylenglykol, Diethylenglykol; Triethylenglykol; Tetraethylenglykol; Dipropylenglykol; Tripropylenglykol; Butylenglykol; 1,3-Butandiol; Methylpropanediol; Pentylenglykol; Hexamethylenglykol; Cyclohexandimethanol; 2,2,4,4-Tetramethyl,-1,3-Cyclobutanediol; Neopentylglykol; PEG-4 bis -100; PPG-9 bis -34.

6. Zusammensetzung nach Anspruch 5, wobei das Glykol Trimethylenglykol ist.

7. Zusammensetzung nach einem der Ansprüche 1 bis 6, wobei die eine kationische Spezies oder die mehreren kationischen Spezies quaternäre Ammoniumpolymere sind.

8. Zusammensetzung nach Anspruch 7, wobei die kationische Spezies Polyquaternium-47 ist.

9. Kosmetisches Präparat zur Hautpflege, umfassend die Zusammensetzung nach einem der vorangehenden Ansprüche.

10. Kosmetisches Präparat nach Anspruch 9, wobei das kosmetische Präparat zum Behandeln von Axelhöhlen verwendet wird.

11. Kosmetisches Präparat nach Anspruch 9, wobei das kosmetische Präparat zum Behandeln von Füßen verwendet wird.

12. Prozess zum Herstellen der Zusammensetzung nach einem der Ansprüche 1 bis 8, wobei der Prozess die folgenden Schritte umfasst:
- Hinzufügen von wasserlöslichen Komponenten zu Wasser, um ein erstes festes Gemisch zu erzeugen, und Erhitzen des ersten Gemisches auf 75 °C;
- Mischen von Fettkomponenten in ein zweites Gemisch und Erhitzen des zweites Gemisches auf 80 °C;
- Hinzufügen des zweiten Gemisches zu dem erhitzten ersten Gemisch, um ein drittes Gemisch zu erzeugen;
- Emulsifierenlassen des dritten Gemisches und Abkühlenlassen davon auf 40 °C;
- Gegebenenfalls Hinzufügen weiterer Inhaltsstoffe zu der Emulsion; und
- Abkühlenlassen der Endemulsion auf 30 °C.

13. Verwendung der Zusammensetzung nach einem der Ansprüche 1 bis 8 zum Herstellen eines kosmetischen Präparats zur Hautpflege.

## Revendications

1. Composition antiperspirante/déodorante aqueuse comprenant, sur la base du poids total de la composition, 0 % en poids de cations aluminium, un ou plusieurs glycols ou polyglycols, et au moins 5 % en poids d'un ou plusieurs sels d'halogénure ionisables solubles dans l'eau, dans laquelle les un ou plusieurs sels d'halogénure sont des agents antimicrobiens, et dans laquelle la composition a une plage de pH de 2 à 8, dans laquelle les un ou plusieurs sels d'halogénure sont choisis dans le groupe constitué du chlorure de sodium et du chlorure de potassium, dans laquelle ladite composition comprend en outre une ou plusieurs espèces cationiques non métalliques solubles dans l'eau.

2. Composition selon la revendication 1, dans laquelle la concentration des un ou plusieurs sels d'halogénure est de 5 à 35 %, de préférence de 5 à 25 %, et de manière davantage préférée de 10 à 25 % en poids de la composition totale.

3. Composition selon l'une quelconque des revendications 1 et 2, dans laquelle la concentration des un ou plusieurs glycols ou polyglycols est de 0,5 à 50 %, de préférence de 1 à 15 % en poids de la composition totale.

4. Composition selon l'une quelconque des revendications 1 à 3, dans laquelle la concentration des une ou plusieurs espèces cationiques est de 0,01 à 10 %, de préférence de 0,05 à 0,5 % en poids de la composition totale.

5. Composition selon l'une quelconque des revendications précédentes, dans laquelle les un ou plusieurs glycols ou polyglycols sont choisis dans le groupe constitué du glycérol; du diglycérol ; du triméthylène glycol ; du tétraméthylène glycol ; de l'éthylène glycol ; du diéthylène glycol ; du triéthylène glycol ; du tétraéthylène glycol ; du dipropylène glycol ; du tripropylène glycol ; du butylène glycol ; du 1,3-butanediol; du méthylpropanediol; du pentylène glycol ; de l'hexaméthylène glycol ; du cyclohexanediméthanol ; du 2,2,4,4-tétraméthyl-1,3-cyclobutanediol ; du néopentyl glycol ; PEG-4 à 100 ; PPG-9 à 34.

6. Composition selon la revendication 5, dans laquelle le glycol est le triméthylène glycol.

7. Composition selon l'une quelconque des revendications 1 à 6, dans laquelle les une ou plusieurs espèces cationiques sont des polymères d'ammonium quaternaire

8. Composition selon la revendication 7, dans laquelle l'espèce cationique est le Polyquaternium-47.

9. Préparation cosmétique pour un soin de la peau comprenant la composition selon l'une quelconque des revendications précédentes.

10. Préparation cosmétique selon la revendication 9, dans laquelle la préparation cosmétique est utilisée pour le traitement des aisselles.

11. Préparation cosmétique selon la revendication 9, dans laquelle la préparation cosmétique est utilisée pour le traitement des pieds.

12. Processus de préparation de la composition selon l'une quelconque des revendications 1 à 8, lequel processus comprend les étapes consistant à
- ajouter des composants hydrosolubles dans l'eau pour faire un premier mélange et chauffer le premier mélange à 75 °C ;
- mélanger des composants gras dans un deuxième mélange et chauffer le deuxième mélange à 80 °C ;
- ajouter le deuxième mélange au premier mélange chauffé pour faire un troisième mélange ;
- laisser le troisième mélange s'émulsionner et refroidir à 40 °C ;
- ajouter facultativement d'autres ingrédients dans l'émulsion ; et
- laisser l'émulsion finale refroidir à 30 °C.

13. Utilisation de la composition selon l'une quelconque des revendications 1 à 8 pour la préparation d'une préparation cosmétique pour un soin de la peau.
